# EUROPEAN PATENT SPECIFICATION

(11) **EP 0 433 067 B1**
(45) Date of publication and mention of the grant of the patent: **29.03.1995**
(21) Application number: 90313579.6
(22) Date of filing: 13.12.1990
(51) Int. Cl.: A61K 47/48

(54) **A non-specific immunomodulating agent and a process for its production**
Nichtspezifisches Immunomodulatormittel und Verfahren zu dessen Herstellung
Agent immunomodulateur non spécifique et son procédé de préparation

(30) Priority: 13.12.1989 GB 8928160
(43) Date of publication of application: 19.06.1991
(73) Proprietor: Büyükkoca, Edip, Prof. Dr., Sisli-Istanbul (TR)
(72) Inventor: Büyükkoca, Edip, Prof. Dr., Sisli-Istanbul (TR)
(74) Representative: Coleiro, Raymond

(56) References cited:
- WO-A-90/01957
- US-A- 4 305 390
- J. CHEM. PHYS., vol. 84, no. 3, 1st February 1986, pages 1443-1450, American Institute of Physics, New York, US; W.H. BRECKENRIDGE et al.
- J. PHYS. CHEM., vol. 92, 1988, pages 4574-4576, American Chemical Society; J.P. VISTICOT et al.

## Description

This invention relates to a non-specific immunomodulator comprising a compound, usually itself having biological activity, believed to be in the form of a complex, and to a process for its production. This process makes use of a technique in which a laser vaporisation source is coupled to a supersonic expansion.

Breckenridge et al, J. Chem. Phys. (1986), 84(3), 1443-1450 describe the formation of Hg-H₂ van der Waals complexes. These are prepared in a supersonic jet by the expansion of a mixture of He, Hg and H₂. The resultant complex is formed in an expansion chamber at a final total pressure in the region of 1 Torr. The complex is then excited with laser radiation so as to generate, selectively, an eletronically excited state of the complex which can produce chemical products.

Visticot et al, J. Phys. Chem. (1988), 92, 4574-4576 (also abstracted in Chemtracts - Analytical and Physical Chemistry, Jan/Feb 1989) describe the excitation of a Ca/HCl complex in the vicinity of a calcium rod and the subsequent excitation of the complex to form CaCl in either the A or B state. Thus, a mixture of argon and HCl is introduced into a channel in which is disposed a rotating Ca rod on which is focused a Nd:YAG laser so as to produce predominantly ground-state Ca atoms. The mixture of argon, HCl and Ca is then expanded into a vacuum, whereupon the cooling due to expansion causes the Ca to react with the HCl and thereby form copious quantities of Ca/HCl complexes. The Ca atoms in the complexes is excited by a second laser, resulting in the formation of CaCl in either the excited A or B state. This can be detected as a result of the emission of fluorescence.

To date, these processes have not been employed for production of compounds having biological activity, especially anti-neoplastic and anti-tumor agents which are effective in influencing the immuno system of the body.

It is believed that, in the absence of any treatment, the immuno response system of the body operates, in response to the presence of cancer cells, as follows. The genetic code of cancer cells is recognised by T-lymphocites, which in response generate the necessary immuno reactions to prevent or inhibit further growth. Usually, in order to achieve this function, the T-lymphocite cells elicit a response from B-lymphocites which assist in this inhibiting action. However, because cancer cells affect the normal genetic code, the T-lymphocites do not always recognise the genetic code of the cancer cells and so do not identify their presence. Accordingly, the immuno system breaks down and the cancer cells proliferate.

In the past, Rosenberg has attempted to isolate the T-lymphocites from the body, modify them so that they are able to recognise the change in the genetic code resulting from the presence of the cancer cells and trigger a response by the B-lymphocites, thereby inhibiting growth of cancer cells.

However, this method has the difficulty that too little a modification will not be effective, whereas too drastic a modification will cause the T-lymphocites to elicit a response which will kill not only cancer cells but normal cells. This may be fatal to the patient.

Surprisingly, I have now found an agent, believed to be a van der Waals complex, which on administration acts as a non-specific immunomodulator and which will not only prevent further growth of cancer cells but may also remove neoplastic cells and indeed tumors caused thereby without adversely affecting normal living cells.

The immunomodulating agent comprises an acid or acid salt, and more preferably an acidic compound which itself has biological activity, the molecule of which compound is held in an excited stated, believed to be in the form of a complex which may contain oxygen (which may be provided by oxygen and/or ozone), and contains halogen, especially chlorine, and a metal.

Such compunds are to be contrasted with mere conventional diagnostic agents containing phosphorescent markers. In such a case, although the marker might be regarded as being excited, the biologically active component remains unexcited.

The active compound is believed to be the biologically active component of the complex. However, when in its excited state, it has quite remarkably different biological activity as compared with the compound when in its normal stable state and free from the complex. In particular, the compound behaves as an immunomodulator which may exhibit excellent neoplastic, and more particularly anti-tumor, activity.

The compound is brought to its excited state using a laser vaporisation source followed by a supersonic expansion, for example, as described by Visticot et al, supra.

It is believed that the compound remains chemically unaltered within a complex and suffers only a change in electron density so that is assumes an excited state.

The components of the complex are apparently held together by weak attractive forces, probably van der Waals' forces, acting between molecules. Such forces are somewhat weaker than hydrogen bonds and far weaker than interatomic valences. They probably arise from the interaction of the electron cloud or field of one molecule with that of another which occurs when the molecules are close together. It was suggested by London (1930) that in molecules (and atoms) of all kinds, the positively charged nuclei and the negatively charged electrons must be regarded as undergoing some kind of oscillation with respect to one another; as a result of this displacement of positive and negative charges, every molecule behaves as an oscillating dipole. It is the electrostatic attraction between these dipoles and corresponding dipoles induced in adjacent molecules that is the cause of such molecular attraction. Although not wishing to be bound by theory, it is believed that such bonding holds the complex together.

The active component incorporated in the complex may be any acid or acid salt compound capable of forming such a complex with a vaporisable substance, oxygen (or ozone) and a halogen atom.

The complex may be represented by

MX/Ac/(O)ₘ

where Ac is the active ingredient,
M is a metal provided by the vaporizable substance,
X is a halogen atom or a mixture thereof, and
m represents the number of oxygen atoms and is at least 1, possibly 2, preferably at least 3 and more preferably 3.

The active component may be any acid or acidic salt capable of forming the above complex, but especially preferred are aromatic (optionally aryl substituted) carboxylic acids having, adjacent a carboxyl group, an optionally substituted carboxyl or acyloxy group.

However, in addition to such compounds, of which a complex containing 2-(acetyloxy)benzoic acid is most preferred, examples of the acid or acidic salt are boric acid, acetic acid, citric acid, lactic acid, L-ascorbic acid, sodium bicarbonate, potassium bicarbonate, aluminium hydrogen sulphate, alkali, especially sodium or potassium, mono-and diphosphate salts, DNA, RNA, chitin, glutamic acid, vitamin A, vitamin B, the mono and diphosphate salts of vitamin B, vitamin E, the acetate and succinate esters of vitamin E, quinine and penicillin, derivatives of quinine and penicillin such as a semi-penicillin, ibuprofen, opiates, polysaccharides, polypeptides (especially proteins in an acid medium), lauric acid, stearic acid and dodecylbenzene sulphonic acid.

The halogen atom X may be chlorine, iodine, bromine or fluorine and the vaporizable substance may be vaporizable substance capable of providing a metal, especially an alkali metal, for example sodium, potassium or a mixture thereof, capable of reaction with the halogen.

However, the vaporizable substance is preferably a halogen salt, more preferably of a metal, especially an alkali metal, for example NaCl, NaI, NaBr, NaF, KCl, KI, KBr, KF, ZnCl₂, ZnI₂, ZnBr₂, ZnF₂, CuCl₂, CuI₂, CuBr₂, CuF₂, MgCl₂, MgI₂, MgBr₂ and MgF₂. Most preferred are KCl, NaCl and mixture thereof, especially a single crystal of KCl, NaCl or K/Na/Cl.

Even when the vaporizable substance is a halogen salt providing halogen, additional halogen should be provided by elemental halogen. In such a case, the halogen of the salt is preferably the same as the elemental halogen.

Although the active component is usually an acidic compound, the pH of the final complex is usually in the range of 5.5-7.5 inclusive.

The complex may contain more than 1 mole of active component, and preferably contains 2, or in some cases 3.

A complex in which the active component is acetic acid may contain 3 moles of active component.

Where, as mentioned above, the active component is an aromatic, especially phenyl, carboxylic acid having, adjacent a carboxyl group, an optionally substituted carboxyl or acyloxy group, it is believed that a typical complex (for a 2-acyl substituted benzoic acid) may have a structure represented by 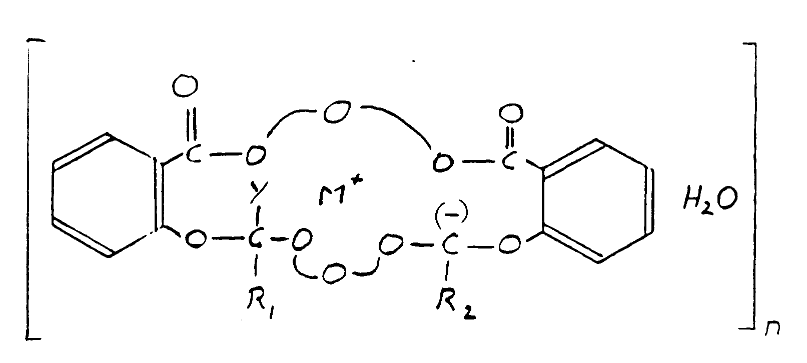
wherein R₁ and R₂ are, independently of one another, hydrogen, an optionally substituted, saturated or unsaturated, straight or branched chain aliphatic hydrocarbon radical (preferably a C₁₋₄ alkyl group such as methyl or propyl), or amino, alkylamino, dialkylamino, acylamino, alkoxy, acyloxy, hydroxy, aryloxy, nitro, sulphur, methylsulphonylamino, mercapto, allylmercapto, carboxy, carboxyalkyl or carboxamido, M is any element having a positive valency, preferably a metal, more preferably an alkali metal atom, Y is halogen, alkyl (preferably a C₁₋₄ alkyl group such as methyl, ethyl or propyl), haloalkyl or haloaryl, and n is at least 1, but is usually a high number, perhaps say up to 1000 or more. The aryl group or moiety of R₁, R₂ and Y is preferably an optionally substituted phenyl.

Although the complex is shown as having two benzene rings, it may have more.

When the active ingredient is the preferred 2-(acetyloxy)benzoic acid, it is believed that the complex has the formula: 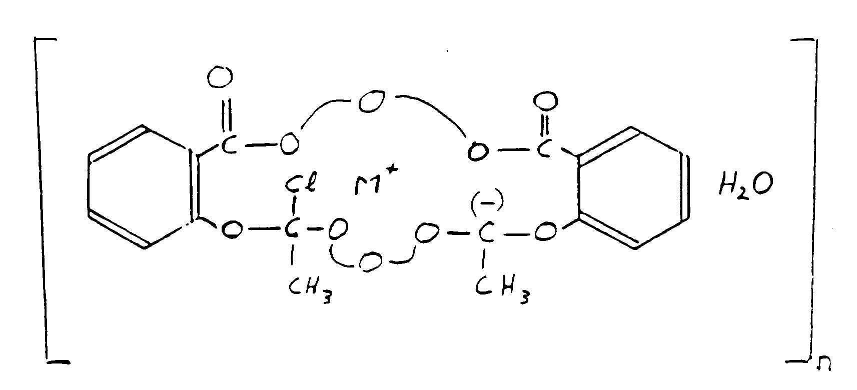
In an especially preferred product, the alkali metal ion M₁ is K, Na or a mixture thereof.

I have isolated the following products:
1. A product in which the alkali metal ion M⁺ is K⁺, which product has an empirical formula which may be represented by C₁₈H₁₆O₁₁ ClK believed to be C₁₈H₁₄O₁₀KCl.H₂O (complex with 2 molecules of acetyl salicylic acid), a molecular weight of 482.82g/mole and contains C 44.737%; H 3.313%; O 36.452%; Cl 7.352%; K 8.077%. Its production may be represented by

   [KCl/2(C₉H₈O₄)]+O₃ +Cl₂ +He→C₁₈H₁₄O₁₀.KCl.H₂O

   or [½KCl/C₉H₈O₄]+O₃ +Cl₂ +He → C₉H₇O₅.½KCl.H₂O

   or KCl/aspirin+O₃ +Cl₂ +He → Edoferon kappa (see below)

   From a rough estimation, its molecular weight appeared to be in the region of about 450-500k daltons. This product is especially preferred and I call it "Edoferon kappa".
2. A product in which the alkali metal ion M⁺ is Na⁺, which product has an empirical formula C₁₈H₁₆O₁₁ClNa, believed to be C₁₈H¹₄O₁₀NaCl.H₂O. From a rough estimation, its molecular weight appeared to be in the region of about 400-450kd. I call this product "Edoferon natilus".
3. A complex containing citric acid, potassium, chlorine and oxygen. I call this product "Edoferon KB".

An immunomodulating agent in accordance with the invention may be prepared using a technique, such as that described by Visticot et al, supra, in which a laser vaporisation source is coupled to a pulsed supersonic free jet expansion. In particular, the processing may comprise the steps of incorporating an active component in a mixture of oxygen and/or ozone, halogen (preferably chlorine) and an inert gas, such as Kr, Ar, Ne, N₂ and most preferably He, under pressure, which mixture is preferably liquid under the said pressure, directing the mixture containing the active component at a rotating body of a substance capable of vaporisation, directing at the rotating body a laser so as to effect said vaporisation, and allowing the said mixture and the vaporised substance to expand into a vacuum and thereby form a complex, whereby the active component assumes an excited state.

Preferably, the process includes the additional steps of exciting the complex with a second laser.

When the substance capable of vaporization is an alkali metal salt, the rotating body preferably comprises a crystal, more preferably a single crystal.

Preferably the mixture of the active component in oxygen and/or ozone, halogen and the inert gas under pressure is directed at the rotating crystal in pulses.

I have found that the process is particularly suitable for producing complexes containing boric acid, citric acid, L-ascorbic acid, sodium and potassium bicarbonate, mono- and disodium phosphate, vitamin A, a semi-penicillin, ibuprofen, lauric acid, stearic acid and dodecylbenzene sulphonic acid.

In a particularly preferred process, the immunomodulating agent, believed to be a van der Waals adduct, is prepared in a pulsed supersonic free jet expansion apparatus using, as the expansion gas, a mixture of He, O₃ and Cl₂ in which the active component is present. Molecules of an alkali metal salt, especially potassium chloride, are introduced into the expansion chamber by laser oblation of a single crystal of the alkali metal salt on which is focused a laser, especially a Nd:YAG laser as described in Visticot et al, supra. The resultant complex, which may be represented by MX/Ac, for example KCl/C₉H₈O₄ (aspirin), is excited by a second laser beam in the presence of the expansion gas and forms the final complex, containing the active component in an excited state. The final complex may be purified, preferably using supercritical fluids extraction.

Typically, in the final product the alkali metal salt will provide 1-25% by weight of the total complex.

The immunomodulatory agent in accordance with the invention may be administered alone or in combination with a suitable excipient. They may be administered orally or parenterally and typical dosage forms are as a powder, tablet, capsule, suspension or solution, especially an isotonic solution which may be freeze dried.

Typically a solution may contain from 0.5g/l up to a saturation concentration which may be say 2.5g/l, and preferably contains 1-2g/l. For Edoferon kappa a preferred concentration is 1.2g/l.

A solid composition may, for example, contain up to 80 or even up to 98% of the active ingredient.

The shelf-life of the immunomodulatory agent may be about 1 year.

As will be explained in more detail below, especially when administering the complexes which I call Edoferon kappa and Edoferon natilus, the immunomodulatory and anti-neoplastic activity are so great that is it not necessary to employ statistical methods for assessment of the rate of destruction of benign cellular material; its destruction may be readily observed visually.

Indeed, substantially total destruction may be observed within a period of about 3 weeks.

Excellent immunomodulatory activity has also been achieved using complexes of sodium or potassium chloride, chlorine, oxygen and, in place of 2-(acetoxy) benzoic acid, boric acid, citric acid, L-ascorbic acid, sodium and potassium bicarbonate, the semi-penicillin commercially available as Longatran (Bayer AG) and ibuprofen (a non-steriod anti-inflammatory agent).

In particular, trials using Edoferon kappa and natilus have shown that this has important immunomodulatory and in particular, anti-neoplastic activity against mice carcinoma and more especially Ehrlich Ascites Carcinoma. Furthermore, Edoferon may well be capable of inhibiting a recurrence of such carcinoma and prolong survival of humans with, eg, melanoma, bladder cancer, aesophagus and stomach carcinomas, head and neck tumors and leukaemia.

Whatever the theory, it would appear that application of Edoferon provides effective results by virtue of three successive stages, namely (a) production of one tumor infiltrated lymphocite, (b) reproduction of many such T-lymphocites which are then capable of infiltration into the tumor cells (during which time the tumor still remains and resembles an ulceric cell), and (c) burning of the cells so as to remove the tumor. Thus, after Edoferon treatment, any tumor cell on the skin may be completely removed leaving little or no trace. Likewise, the Edoferon may act upon benign growth with similar effect.

The invention will now be described in more detail with reference to the following examples and accompanying drawings, in which
Fig. 1 illustrates an IR spectrum of 2-(acetyloxy)benzoic acid in its conventional form,
Fig. 2 is the IR spectrum of Edoferon kappa and
Fig. 3 is a graph illustrating the survival rates of EAC-transplanted BABL/c mice treated with Edoferon kappa (see the Experimental studies below).

### Preparation of Edoferon kappa

### Preparation Example

2.4 moles of acetyl salicylic acid (aspirin) are introduced into a pressure chamber containing liquid helium (5 moles), ozone (1 mole) and chlorine (1 mole). The pressure chamber is equipped with a valve which when open allows the reaction mixture to expand into a 1mm diameter channel. About 1cm downstream, it reaches a rotating crystal of potassium chloride (1 mole) on which an Nd:YAG laser is focused and which vaporized the crystal. Typically, the crystal will weigh about 1g. Then the mixture of KCl, He, ozone and chlorine are expanded into a vacuum where, due to cooling by the expansion, a complex is formed. The beam of a second laser crosses the molecular beam about 15mm downstream. The second pulse is delayed with respect to the first by approximately 30»s corresponding to the time taken by the supersonic stream of potassium chloride to reach an observation region. This second laser excites the resulting molecular beam so as to form the desired complex. A typical pressure in the expansion chamber is about 0.01 Torr.

The resultant product, Edoferon kappa, is purified by extraction, under supercritical conditions, with a fluid, for example CO₂/methanol (75/25) in which at least a portion of the methanol is optionally replaced by isopropyl alcohol.

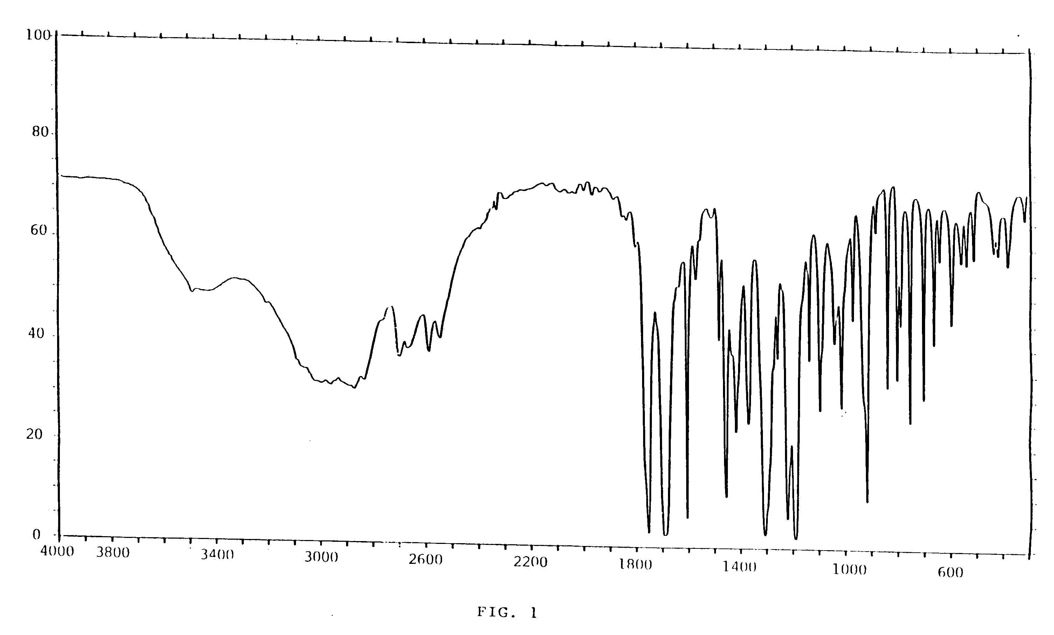

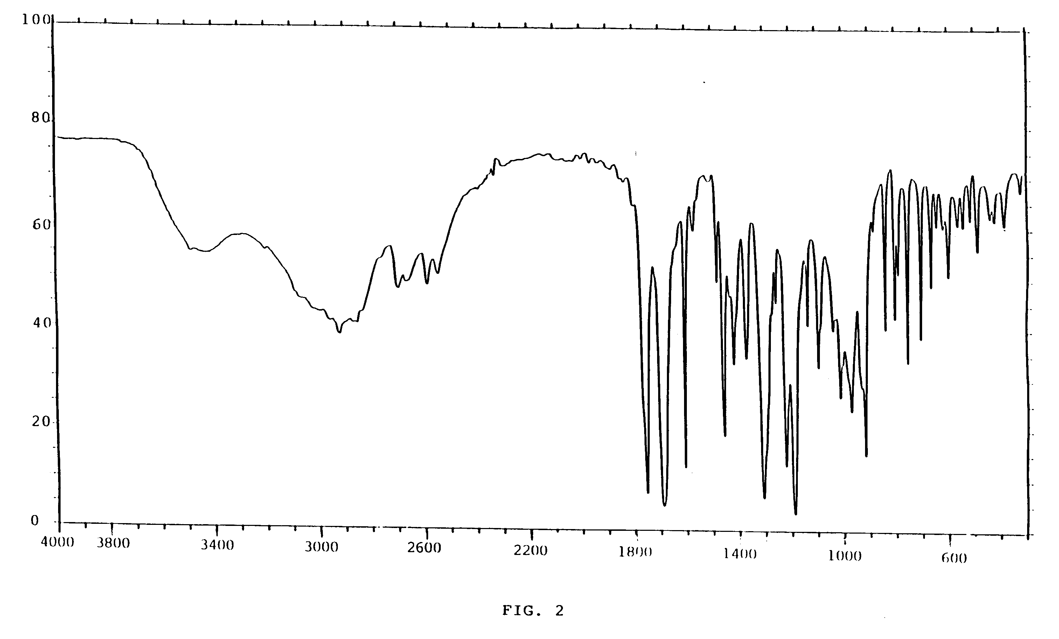

As can be seen from a comparison of Figs. 1 and 2, the chemical structure of the Edoferon kappa complex is essentially the same as that of a conventional acetyl salicylic acid when assessed by IR spectroscopy. However, as a result of the above-mentioned process, the molecules of the acetyl salicylic acid thus produced are in an excited state and are apparently bound to oxygen and chlorine by van der Waals' forces.

The Edoferon kappa is slightly soluble in water and has greater solubility in some alcohols such as methanol.

It has a shelf-life of at least one year.

The corresponding sodium compound, Edoferon natilus, can be prepared in the same manner using a single sodium chloride crystal in place of the potassium chloride.

### Experimental Studies

### 1. In Vitro Direct Cytotoxicity of Edoferon on Various Tumour Cells.

The effect of Edoferon (KA,KB and NA forms) on eukaryotic cell growth was studied with the use of the neutral-red dye uptake assay. The following tumour cell lines were used: LT5.1.SK23, A375,DX3 (Melanoma),SW742 (colon carcinoma) and MCF7,ZR75,T47D (breast carcinoma).

In particular, the tumour cells (5 x 10⁴/mL) were grown in 96 well plates in serum containing medium into which EDOFERONs (KA,KB and NA) were added in concentrations decreasing in a three-fold dilution range, ie concentrations decreasing within a range of from 1mg/ml down to 0.001 mg/ml. Following 72h. incubation at 37°C in 5% CO₂ atmosphere, cells were stained with 0.3% neutral red dye (in 0.008 mM phophate buffer) for 45 mins. Optical density (O.D 550 nm.) of eluted dye was determined, and data expressed as a percentage of untreated control cell preparation for each cell type.

The results were represented by graphs of survival rates against drug concentration and the survival rates given represented a mean of octuplicate readings of optical densities of the dye which had been taken up by the cells at the given drug concentrations. For reasons which are not easily explainable (possibly due to increased membrane permeability) the cells treated with the drugs (usually at sub-toxic concentration) take up more dye than the untreated control cells, thus giving slightly higher optical density values. Because of this, some of the survival rate values obtained at low concentrations were slightly greater than 100%.

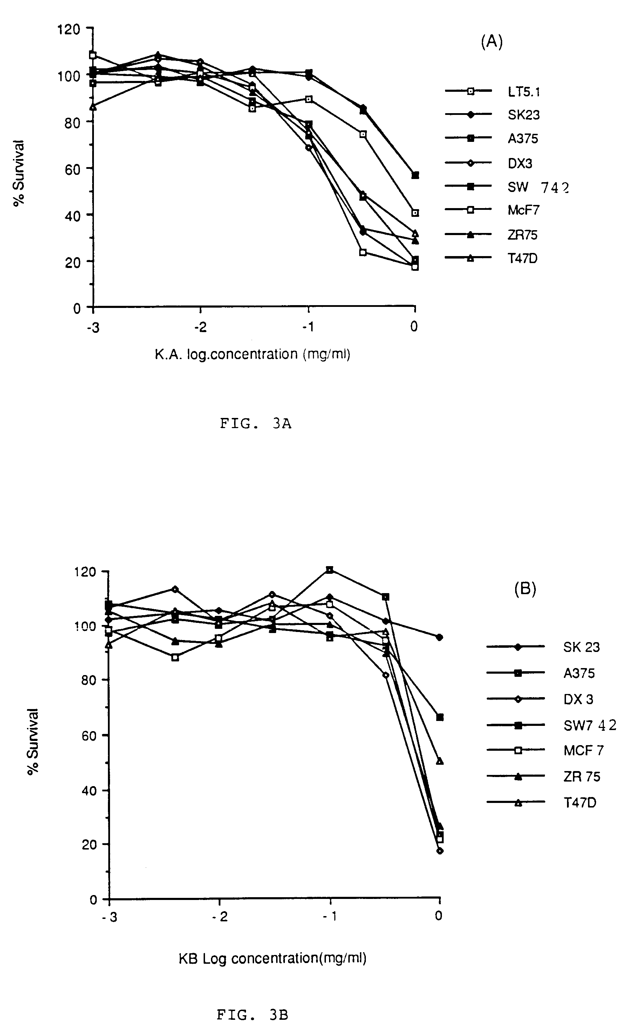

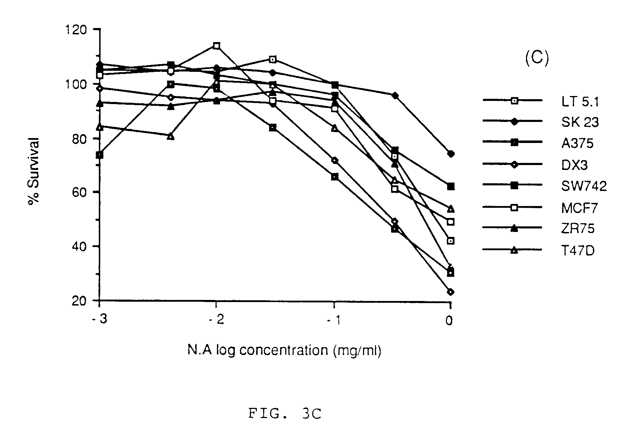

The results are presented in Figures 3A-C for EDOFERON KA,KB and NA respectively and represent the dose-dependent inhibition of tumour cell proliferation expressed as a percentage of untreated controls. The degree of inhibition varied with the cell types, and was minimal for the LT5.1,SK23 and SW742 lines. In contrast MCF7,DX3,ZR75,A375 and T47D tumour cell lines showed only 23-48% survival in the presence of 0.33 mg/mL of EDOFERON-KA for 72 hours (Fig. 1A). At the same concentration, however, neither EDOFERON-B or NA showed any appreciable growth inhibition effect against the majority of tumour cell lines, with the exception of A375 and DX3 lines (47 and 50% survival, respectively) treated with EDOFERON-NA.

In view of the above it is concluded that EDOFERON-KA is a good canditate for in-vivo studies and its anti-tumourigenic activity should be evaluated further.

### 2. In vivo tests in Mice

Lethal dose tests in mice have shown that oral administration of Edoferon kappa in mice gives an LD₅₀ > 50,000mg/kg, though its specific LD₅₀ was not measured in this test.

In the laboratories of the Center for Experimental Medical Research and Application of Istanbul University, the anti-tumor and immunomodulatory effects of Edoferon have been tested on mice bearing Ehrilich Ascites Carcinoma (EAC) solid tumor. Adult mice of Balb/c, weighing approximately 20g and bred at the same laboratory, were used in this study. The animals were fed ad libitum with a standard laboratory diet, purchased from Istanbul Yem Sanayi Topkapi-Istanbul, and water. EAC was obtained from a male mouse into which the ascites tumor had been implanted intraperitoneally 14 days previously.

Fifteen animals were inoculated with 1.6 x 10⁷ Ehrlich ascites (0.2ml liquid medium for each mouse) tumor cells subcutaneously in their left hind limb. Following the week after inoculation, mice bearing tumors of considerable size were arranged in the following respective groups: (a) a control group consisting of mice which did not receive any therapy, (b) mice of a so-called "I.Edoferon" group which were fed Edoferon with tap water at a dose of 0.8mg/g per day, and (c) mice of a so-called "II.Edoferon" group which were fed Edoferon with tap water at a dose of 1.6mg/g per day.

On the first day of the experiment, 0.2ml of Ehrlich ascites fluid containing approximately 1.6 x 10⁷ cells was administered subcutaneously to each mouse. On the eighth day, Edoferon was administered to the animals of the second and third groups in the respective dosage mentioned above.

### Results

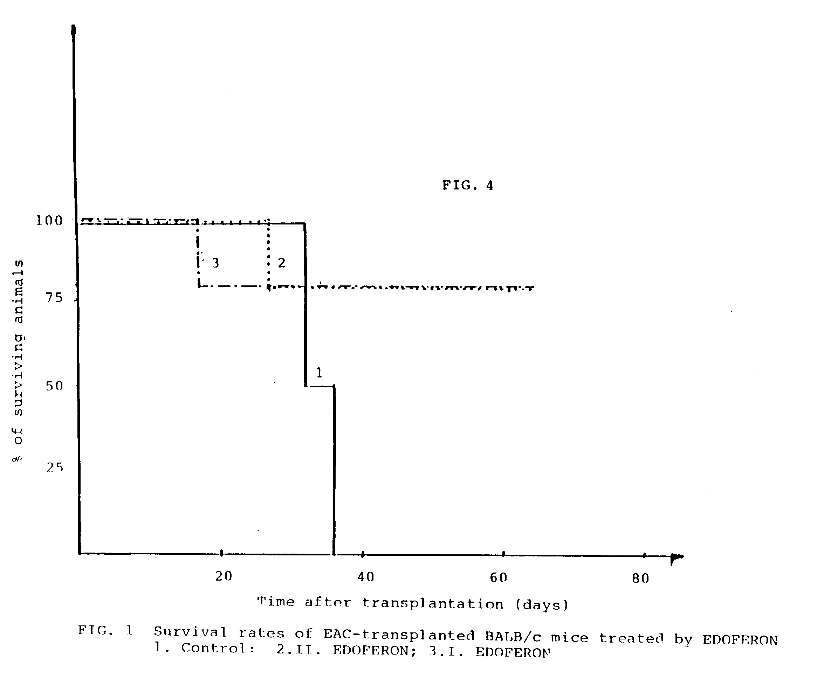

The survival rates are shown in Fig. 4. The longest survival time of any animal of the control group was 31 days. In contrast, the % of surviving animals was 80 for both the second and third groups (the II. and I. Edoferon groups respectively) at the end of the 64th day of the experiment; in each of the second and third groups, one accidental death occurred.

This study shows that Edoferon kappa has both superior anti-tumor and immunomodulatory effects at each of the doses 0.8mg/g and 1.6mg/g in mice. Inhibition of tumor growth was observed after 6 days from the beginning of Edoferon treatment, while complete loss of the tumours was observed at 21 days from the beginning of the Edoferon treatment.

### 2. Application of Edoferon to Non-Malignant Mole on Human skin

The anti-neoplastic effect of Edoferon kappa upon a non-malignant mole above the inventors' right eyebrow was observed after injecting into the mole 1ml of a 0.15mg/ml isotonic solution of Edoferon.

### Results

Before treatment, a prominant mole was visible. After 6 days from the Edoferon kappa injection, the mole became inflamed but after 2 weeks had diminished considerably. After 3 weeks, the mole was even less prominant.

### Conclusions

It can be seen from the above in-vivo tests conducted on mice and those conducted on the inventor himself that the anti-tumour and immunomodulatory effects of Edoferon kappa are so great as to be capable of visual assessment without the need to perform the statistical calculations and minute size change measurements which are conventionally necessary.

It seems that the cells, especially malignant cells, first become inflamed and then are burned by the Edoferon treatment over a period of about 3 weeks. This behaviour is similar to that observed after application of a smallpox vaccine.

This observation provides a new line for cancer research and it is likely Edoferon will be useful as a carcinoma vaccine for several types of cancer.

It exhibits two functions, one being immunomodulatory and the other anti-neoplastic.

It is expected also that the drug will have an antiinflammatory (including antiarthritic) and cholesterol level controlling effect upon patients suffering from hypo or hyper cholesterol symptoms.

Whatever the theory underlying this phenomenon, it is clear that a complex embodying the invention is likely to produce a useful medicament of particular importance in the field of cancer research.

## Claims (Claims for the following Contracting State(s): AT, BE, CH, DE, FR, GB, IT, LI, LU, NL, SE)

1. An immunomodulating agent having biological activity and comprising at least one molecule of an active component, which active component is an acid or acid salt bound, by van der Waals' type forces, within a complex, together with a metal and a halogen atom, and which active component is held in an excited state created by laser vaporization followed by supersonic expansion.

2. An agent according to claim 1 containing at least two molecules of the active component.

3. An agent according to claim 1 or claim 2, wherein the complex additionally contains an oxygen atom.

4. An agent according to any preceding claim, wherein the halogen atom is chlorine.

5. An agent according to claim 3 or claim 4, wherein the acid complex has the formula
MX/Ac/(O)ₘ
where Ac is the active component,
M is a metal provided by a vaporizable substance,
X is a halogen atom or a mixture thereof, and
m represents the number of oxygen atoms and is at least 1.

6. An agent according to claim 5, wherein M is an alkali metal.

7. An agent according to claim 5 or claim 6, wherein m is 3.

8. An agent according to any preceding claim, wherein the active component is an acid.

9. An agent according to any one of claims 1 to 7, which is a partial salt of an inorganic or organic acid.

10. An agent according to any one of claims 1 to 9, in which the active component is boric acid, acetic acid, citric acid, L-ascorbic acid, sodium bicarbonate, potassium bicarbonate, mono- or di-sodium or potassium phosphate, aluminium hydrogen sulphate, DNA, RNA, a polypeptide, glutamic acid, vitamin A, vitamin B or a mono- or di-phosphate salt thereof, vitamin E or an acetate or succinate ester thereof, quinine or penicillin or a derivative thereof, lauric acid, stearic acid, dodecylbenzenesulphonic acid, chitin, lactic acid, a polysaccharide, an opiate, ibuprofen, 2-acetylbenzoic acid or a salt thereof.

11. An agent according to claim 10, in which the compound is boric acid, citric acid, L-ascorbic acid, sodium bicarbonate, potassium bicarbonate, vitamin A, a semi-penicillin, lauric acid, stearic acid, dodecylbenzenesulphonic acid, ibuprofen, or 2-acetyloxybenzoic acid.

12. An agent according to claim 11, which is a complex of 2-acetyloxy(benzoic) acid, potassium, oxygen and chlorine.

13. An agent according to claim 12, which has the empirical formula C₁₈H₁₆O₁₁ClK.

14. An agent according to claim 12 or claim 13, which has a molecular weight of from 450-500 k daltons.

15. An agent according to claim 11, which is a complex of 2-acetyloxy(benzoic) acid, sodium, oxygen and chlorine.

16. An agent according to claim 15, which has the empirical formula C₁₈H₁₆O₁₁ClNa.

17. An agent according to claim 15 or claim 16, which has a molecular weight of from 400-500 k daltons.

18. A compound for use as a pharmaceutical which is an agent according to any preceding claim.

19. A compound for use as a non-specific immuno modulatory agent which is an agent according to any one of claims 1-17.

20. A compound for use as an anti-neoplastic agent which is an agent according to any one of claims 1-17.

21. A compound for use as an anti-tumor agent which is an agent according to any one of claims 1-17.

22. Use of a compound which is an agent according to any one of claims 1-23 for the preparation of a medicament for treatment of benign tissue.

23. A pharmaceutical composition comprising an agent according to any one of claims 1-17 and a pharmaceutically acceptable excipient.

24. A process for preparing a non-specific immunomodulating and/or antineoplastic agent comprising an active component which is an acid or acid salt bound within a complex together with a metal and a halogen atom and present in an excited state, which process comprises incorporating the active component in a mixture of oxygen and/or ozone, halogen and an inert gas under pressure, directing the mixture containing the active component at a rotating body of a substance capable of vaporisation to provide the metal, directing at the rotating body a laser so as to effect said vaporisation, and allowing the said mixture and the vaporised substance to expand into a vacuum and thereby form a complex, whereby the active component assumes an excited state.

25. A process according to claim 24, which includes the additional step of exciting the complex with a second laser.

26. A process according to claim 24 or claim 25, wherein the halogen atom is chlorine.

27. A process according to any one of claims 24 to 26, wherein the inert gas is helium.

28. A process according to any one of claims 24 to 27, wherein the gaseous mixture is in liquid form when under pressure.

29. A process according to claim 28, wherein the liquified gaseous mixture is directed in pulses at the rotating body.

30. A process according to any one of claims 24 to 29, wherein the rotating body comprises a crystal of the said substance capable of vaporization.

## Claims (Claims for the following Contracting State(s): ES, GR)

1. A process for preparing a non-specific immunomodulating and/or antineoplastic agent comprising an active component which is an acid or acid salt bound within a complex together with a metal and a halogen atom and present in an exited state, which process comprises incorporating the active component in a mixture of oxygen and/or ozone, halogen and an inert gas under pressure, directing the mixture containing the active component at a rotating body of a substance capable of vaporisation to provide the metal, directing at the rotating body a laser so as to effect said vaporisation, and allowing the said mixture and the vaporised substance to expand into a vacuum and thereby form a complex, whereby the active component assumes an excited state.

2. A process according to claim 1, which includes the additional step of exciting the complex with a second laser.

3. A process according to claim 1 or claim 2, wherein the halogen is chlorine.

4. A process according to any one of claim 1, claim 2 or claim 3, wherein the inert gas is helium.

5. A process according to any preceding claim, wherein the gaseous mixture is in liquid form when under pressure.

6. A process according to claim 5 wherein the liquified gaseous mixture is directed in pulses at the rotating body.

7. A process according to any preceding claim, wherein the rotating body comprises a crystal of the said substance.

8. A process according to any preceding claim, wherein the said agent contains at least two molecules of the active component.

9. A process according to any preceding claim, wherein the active component is an acid.

10. A process according to claim 9, wherein the active component is a partial salt of the inorganic or organic acid.

11. A process according to claim 10, wherein the complex additionally contains oxygen and halogen atoms.

12. A process according to claim 11, wherein the halogen is chlorine.

13. A process according to claim 12, wherein the acid complex has the formula
MX/Ac/(O)ₘ
where Ac is the active component,
M is a metal provided by a vaporizable substance,
X is a halogen atom or a mixture thereof, and
m represents the number of oxygen atoms and is at least 1.

14. A process according to claim 13, wherein M is an alkali metal.

15. A process according to claim 13 or 14, wherein m is 3.

16. A process according to any one of claims 1 to 7, in which the active component is boric acid, citric acid, L-ascorbic acid, sodium bicarbonate, potassium bicarbonate, vitamin A, a semi-penicillin, ibuprofen or 2-acetyloxybenzoic acid.

17. A process according to any one of claims 1 to 7, in which the active component is an optionally aryl substituted aromatic carboxylic acid having, adjacent a carboxyl group, an optionally substituted carboxyl or acyloxy group.

18. A process according to claim 17, wherein the said agent is a complex comprising 2-acetyloxy(benzoic) acid, sodium, oxygen and chlorine.

19. A process according to claim 18, wherein the said complex has the empirical formula C₁₈H₁₆O₁₁ClK.

20. A process according to claim 18 or claim 19, wherein the said complex has a molecular weight of from 450-500 k daltons.

21. A process according to claim 17, wherein the said agent is a complex comprising 2-acetyloxy(benzoic) acid, sodium, oxygen and chlorine.

22. A process according to claim 21, wherein the said complex has the empirical formula C₁₈H₁₆O₁₁ClNa.

23. A process according to claim 21 or claim 22, wherein the said complex has a molecular weight of from 400-450 k daltons.

24. A process according to any preceding claim, in which the active component has biological activity when in the unexcited state.

25. A process according to claim 24, in which the active component is boric acid, acetic acid, citric acid, L-ascorbic acid, sodium bicarbonate, potassium bicarbonate, mono- or di-sodium or potassium phosphate, aluminium hydrogen sulphate, DNA, RNA, a polypeptide, glutamic acid, vitamin A, vitamin B or a mono- or di-phosphate salt thereof, vitamin E or an acetate or succinate ester thereof, quinine or penicillin or a derivative thereof, lauric acid, stearic acid, dodecylbenzenesulphonic acid, chitin, lactic acid, a polysaccharide, an opiate, ibuprofen, 2-acetylbenzoic acid or a salt thereof.

26. A process according to claim 25, in which the active component is boric acid, citric acid, L-ascorbic acid, sodium bicarbonate, potassium bicarbonate, vitamin A, a semi-penicillin, lauric acid, stearic acid, dodecylbenzenesulphonic acid, ibuprofen, or 2-acetyloxybenzoic acid.

## Patentansprüche (Patentansprüche für folgende(n) Vertragsstaat(en): AT, BE, CH, DE, FR, GB, IT, LI, LU, NL, SE)

1. Immunomodulierendes Mittel mit biologischer Aktivität, das zumindest ein Molekül eines aktiven Bestandteils umfaßt, der eine Säure oder ein saures Salz, gemeinsam mit einem Metall und einem Halogenatom durch Kräfte vom van der Waals-Typ in einem Komplex gebunden, ist, wobei der aktive Bestandteil in einem durch Laserverdampfung gefolgt von Ultraschallexpansion erzeugten angeregten Zustand gehalten ist.

2. Mittel nach Anspruch 1, das zumindest zwei Moleküle des aktiven Bestandteils enthält.

3. Mittel nach Anspruch 1 oder 2, worin der Komplex zusätzlich ein Sauerstoffatom enthält.

4. Mittel nach irgendeinem der vorangegangenen Ansprüche, worin das Halogenatom Chlor ist.

5. Mittel nach Anspruch 3 oder 4, worin der Säurekomplex der Formel
MX/Ac/(O)ₘ
entspricht, worin Ac den aktiven Bestandteil, M eindurch eine verdampfbare Substanz bereitgestelltes Metall, X ein Halogenatom oder ein Gemisch davon und m die Anzahl der Sauerstoffatome, darstellt und zumindest 1 ist.

6. Mittel nach Anspruch 5, worin M ein Alkalimetall ist.

7. Mittel nach Anspruch 5 oder 6, worin m = 3 ist.

8. Mittel nach irgendeinem der vorangegangenen Ansprüche, worin der aktive Bestandteil eine Säure ist.

9. Mittel nach irgendeinem der Ansprüche 1 - 7, das ein partielles Salz einer anorganischen oder organischen Säure ist.

10. Mittel nach irgendeinem der Ansprüche 1 - 9, worin der aktive Bestandteil Borsäure, Essigsäure, Zitronensäure, L-Ascorbinsäure, Natriumbicarbonat, Kaliumbicarbonat, Mono- oder Dinatrium- oder -kaliumphosphat, Aluminiumhydrogensulfat, DNA, RNA, ein Polypeptid, Glutaminsäure, Vitamin A, Vitamin B oder ein Mono- oder Diphosphat-Salz davon, Vitamin E oder ein Acetat- oder Succinatester davon, Chinin oder Penicillin oder Derivat davon, Laurinsäure, Stearinsäure, Dodecylbenzolsulfonsäure, Chitin, Milchsäure, ein Polysaccharid, ein Opiat, Ibuprofen, 2-Acetylbenzoesäure oder ein Salz davon ist.

11. Mittel nach Anspruch 10, worin die Verbindung Borsäure, Zitronensäure, L-Ascorbinsäure, Natriumbicarbonat, Kaliumbicarbonat, Vitamin A, ein Semipenicillin, Laurinsäure, Stearinsäure, Dodecylbenzolsulfonsäure, Ibuprofen oder 2-Acetyloxybenzoesäure ist.

12. Mittel nach Anspruch 11, das ein Komplex aus 2-Acetyloxybenzoesäure, Kalium Sauerstoff und Chlor ist.

13. Mittel nach Anspruch 12, das die empirische Formel C₁₈H₁₆O₁₁ClK besitzt.

14. Mittel nach Anspruch 12 oder 13, das ein Molekulargewicht von 450 - 500 k Dalton besitzt.

15. Mittel nach Anspruch 11, das ein Komplex aus 2-Acetyloxybenzoesäure, Natrium, Sauerstoff und Chlor ist.

16. Mittel nach Anspruch 15, das die empirische Formel C₁₈H₁₆O₁₁ClNa besitzt.

17. Mittel nach Anspruch 15 oder 16, das ein Molekulargewicht von 400 - 500 k Dalton besitzt.

18. Verbindung zur Verwendung als Pharmazeutikum, die ein Mittel nach irgendeinem der vorangegangenen Ansprüche ist.

19. Verbindung zur Verwendung als nichtspezifisches Immunomodulationsmittel, die ein Mittel nach irgendeinem der Ansprüche 1 - 17 ist.

20. Verbindung zur Verwendung als antineoplastisches Mittel, die ein Mittel nach irgendeinem der Ansprüche 1 - 17 ist.

21. Verbindung zur Verwendung als Antitumormittel, die ein Mittel nach irgendeinem der Ansprüche 1 - 17 ist.

22. Verwendung einer Verbindung, die ein Mittel nach irgendeinem der Ansprüche 1 - 23 ist, zur Herstellung eines Medikaments zur Behandlung von gutartigem Gewebe.

23. Pharmazeutische Zusammensetzung, umfassend ein Mittel nach irgendeinem der Ansprüche 1 - 17 und einen pharmazeutisch annehmbaren Exzipienten.

24. Verfahren zur Herstellung eines nichtspezifischen, immunomodulierenden und/oder antineoplastischen Mittels, umfassend einen aktiven Bestandteil, der eine Säure oder ein saures Salz, gemeinsam mit einem Metall und einem Halogenatom in einem Komplex gebunden, ist und der in einem angeregten Zustand vorliegt, wobei dieses Verfahren umfaßt: Einbringen des aktiven Bestandteils in ein Gemisch aus Sauerstoff und/oder Ozon, Halogen und einem Inertgas unter Druck, Leiten des den aktiven Bestandteil enthaltenden Gemisches zu einem rotierenden Körper aus einer Substanz, die in der Lage ist, zur Bereitstellung des Metalls zu verdampfen, Richten eines Lasers auf den rotierenden Körper, um die Verdampfung zu bewirken, und Expandierenlassen des Gemisches und der verdampften Substanz in ein Vakuum, um dadurch einen Komplex zu bilden, wodurch der aktive Bestandteil in einen angeregten Zustand übergeht.

25. Verfahren nach Anspruch 24, das den zusätzlichen Schritt der Anregung des Komplexes mit einem zweiten Laser einschließt.

26. Verfahren nach Anspruch 24 oder 25, worin das Halogenatom Chlor ist.

27. Verfahren nach irgendeinem der Ansprüche 24 - 26, worin das Inertgas Helium ist.

28. Verfahren nach irgendeinem der Ansprüche 24 - 27, worin das gasförmige Gemisch unter Druck in flüssiger Form vorliegt.

29. Verfahren nach Anspruch 28, worin das verflüssigte Gasgemisch pulsweise zum rotierenden Körper geleitet wird.

30. Verfahren nach irgendeinem der Ansprüche 24 - 29, worin der rotierende Körper einen Kristall der verdampfbaren Substanz umfaßt.

## Patentansprüche (Patentansprüche für folgende(n) Vertragsstaat(en): ES, GR)

1. Verfahren zur Herstellung eines nichtspezifischen, immunomodulierenden und/oder antineoplastischen Mittels, umfassend einen aktiven Bestandteil, der eine Säure oder ein saures Salz, gemeinsam mit einem Metall und einem Halogenatom in einem Komplex gebunden, ist, und der in einem angeregten Zustand vorliegt, wobei dieses Verfahren umfaßt: Einbringen des aktiven Bestandteils in ein Gemisch aus Sauerstoff und/oder Ozon, Halogen und einem Inertgas unter Druck, Leiten des Gemisches mit dem aktiven Bestandteil zu einem rotierenden Körper aus einer Substanz, die in der Lage ist, zur Bereitstellung des Metalls zu verdampfen, Richten eines Lasers auf den rotierenden Körper, um die Verdampfung zu bewirken, und Expandierenlassen des Gemisches und der verdampften Substanz in ein Vakuum, um dadurch einen Komplex zu bilden, wodurch der aktive Bestandteil in einen angeregten Zustand übergeht.

2. Verfahren nach Anspruch 1, das den zusätzlichen Schritt der Anregung des Komplexes mit einem zweiten Laser einschließt.

3. Verfahren nach Anspruch 1 oder 2, worin das Halogenatom Chlor ist.

4. Verfahren nach irgendeinem der Ansprüche 1, 2 oder 3, worin das Inertgas Helium ist.

5. Verfahren nach irgendeinem der vorangegangenen Ansprüche, worin das gasförmige Gemisch unter Druck in flüssiger Form vorliegt.

6. Verfahren nach Anspruch 5, worin das verflüssigte Gasgemisch pulsweise zum rotierenden Körper geleitet wird.

7. Verfahren nach irgendeinem der vorangegangenen Ansprüche, worin der rotierende Körper einen Kristall der Substanz umfaßt.

8. Verfahren nach irgendeinem der vorangegangenen Ansprüche, worin das Mittel zumindest zwei Moleküle des aktiven Bestandteils enthält.

9. Verfahren nach irgendeinem der vorangegangenen Ansprüche, worin der aktive Bestandteil eine Säure ist.

10. Verfahren nach Anspruch 9, worin der aktive Bestandteil ein partielles Salz einer anorganischen oder organischen Säure ist.

11. Verfahren nach Anspruch 10, worin der Komplex zusätzlich Sauerstoff- und Halogenatome enthält.

12. Verfahren nach Anspruch 11, worin das Halogenatom Chlor ist.

13. Verfahren nach Anspruch 12, worin der Säurekomplex der Formel
MX/Ac/(O)ₘ
entspricht, worin Ac den aktiven Bestandteil, M ein durch eine verdampfbare Substanz bereitgestelltes Metall, X ein Halogenatom oder ein Gemisch davon und m die Anzahl der Sauerstoffatome darstellt und zumindest 1 ist.

14. Verfahren nach Anspruch 13, worin M ein Alkalimetall ist.

15. Mittel nach Anspruch 13 oder 14, worin m = 3 ist.

16. Verfahren nach irgendeinem der Ansprüche 1 - 7, worin der aktive Bestandteil Borsäure, Zitronensäure, L-Ascorbinsäure, Natriumbicarbonat, Kaliumbicarbonat, Vitamin A, ein Semipenicillin, Ibuprofen oder 2-Acetyloxybenzoesäure ist.

17. Verfahren nach irgendeinem der Ansprüche 1 - 7, worin der aktive Bestandteil eine gegebenenfalls arylsubstituierte, aromatische Carbonsäure ist, die in benachbarter Stellung zur Carboxylgruppe eine gegebenenfalls substituierte Carboxyl- oder Acyloxygruppe besitzt.

18. Verfahren nach Anspruch 17, worin das Mittel ein Komplex aus 2-Acetyloxybenzoesäure, Kalium, Sauerstoff und Chlor, ist.

19. Verfahren nach Anspruch 18, worin der Komplex die empirische Formel C₁₈H₁₆O₁₁ClK besitzt.

20. Verfahren nach Anspruch 18 oder 19, worin der Komplex ein Molekulargewicht von 450 - 500 k Dalton besitzt.

21. Verfahren nach Anspruch 17, worin das Mittel ein Komplex aus 2-Acetyloxybenzoesäure, Natrium, Sauerstoff und Chlor ist.

22. Verfahren nach Anspruch 21, worin der Komplex die empirische Formel C₁₈H₁₆O₁₁ClNa besitzt.

23. Verfahren nach Anspruch 21 oder 22, worin der Komplex ein Molekulargewicht von 400 - 450 k Dalton besitzt.

24. Verfahren nach irgendeinem der vorangegangenen Ansprüche, worin der aktive Bestandteil im nichtangeregten Zustand biologische Aktivität aufweist.

25. Verfahren nach Anspruch 24, worin der aktive Bestandteil Borsäure, Essigsäure, Zitronensäure, L-Ascorbinsäure, Natriumbicarbonat, Kaliumbicarbonat, Mono- oder Dinatrium- oder -kaliumphosphat, Aluminiumhydrogensulfat, DNA, RNA, ein Polypeptid, Glutaminsäure, Vitamin A, Vitamin B oder ein Mono- oder Diphosphat-Salz davon, Vitamin E oder ein Acetat-oder Succinatester davon, (Chinin oder Penicillin oder ein Derivat davon, Laurinsäure, Stearinsäure, Dodecylbenzolsulfonsäure, Chitin, Milchsäure, ein Polysaccharid, ein Opiat, Ibuprofen, 2-Acetylbenzoesäure oder ein Salz davon ist.

26. Verfahren nach Anspruch 25, worin der aktive Bestandteil Borsäure, Zitronensäure, L-Ascorbinsäure, Natriumbicarbonat, Kaliumbicarbonat, Vitamin A, ein Semipenicillin, Laurinsäure, Stearinsäure, Dodecylbenzolsulfonsäure, Ibuprofen oder 2-Acetyloxybenzoesäure ist.

## Revendications (Revendications pour l'(les) Etat(s) contractant(s) suivant(s): AT, BE, CH, DE, FR, GB, IT, LI, LU, NL, SE)

1. Agent d'immunomodulation ayant une activité biologique et comprenant au moins une molécule d'un composant actif, lequel composant actif est un acide ou un sel d'acide lié, par des forces du type van der Waals, dans un complexe, ensemble avec un métal et un atome d'halogène, et lequel composant actif est maintenu dans un état excité créé par une vaporisation laser suivie par une expansion supersonique.

2. Agent selon la revendication 1 contenant au moins deux molécules du composant actif.

3. Agent selon la revendication 1 ou 2, dans lequel le complexe contient additionnellement un atome d'oxygène.

4. Agent selon l'une quelconque des revendications précédentes, dans lequel l'atome d'halogène est le chlore.

5. Agent selon la revendication 3 ou 4, dans lequel le complexe acide a la formule
MX/Ac/(O)ₘ
où AC est le composant actif,
M est un métal fourni par une substance vaporisable,
X est un atome d'halogène ou un mélange de ceux-ci, et
m représente le nombre d'atomes d'oxygène et est au moins égal à 1.

6. Agent selon la revendication 5, où M est un métal alkali.

7. Agent selon la revendication 5 ou 6, où M est égal à 3.

8. Agent selon l'une des revendications précédentes, où le composant actif est un acide.

9. Agent selon l'une quelconque des revendications 1 à 7, qui est un sel partiel d'un acide inorganique ou organique.

10. Agent selon l'une quelconque des revendications 1 à 9, dans lequel le composant actif est l'acide borique, l'acide acétique, l'acide citrique, l'acide L-ascorbique, le bicarbonate de sodium, le bicarbonate de potassium, le mono- ou di-sodium ou potassium phosphate, l'hydrogèno-sulfate d'aluminium, un ADN, un ARN, un polypeptide, l'acide glutamique, la vitamine A, la vitamine B ou un sel mono- ou di-phosphate de celle-ci, la vitamine E ou un ester acétate ou succinate de celle-ci, la quinine ou la pénicilline ou un dérivé de celles-ci, l'acide laurique, l'acide stéarique, l'acide dodécylbenzènesulfonique, la chitine, l'acide lactique, un polysaccharide, un opiate, l'ibuprofène, l'acide 2-acétylbenzoïque ou un sel de celui-ci.

11. Agent selon la revendication 10, dans lequel le composé est l'acide borique, l'acide citrique, l'acide L-ascorbique, le bicarbonate de sodium, le bicarbonate de potassium, la vitamine A, une semi-pénicilline, l'acide laurique, l'acide stéarique, l'acide dodécylbenzènesulfonique, l'ibuprofène, ou l'acide 2-acétyloxybenzoïque.

12. Agent selon la revendication 11, qui est un complexe de l'acide 2-acétyloxy(benzoïque), le potassium, l'oxygène et le chlore.

13. Agent selon la revendication 12, qui a la formule empirique C₁₈H₁₆O₁₁ClK.

14. Agent selon la revendication 12 ou 13, qui a un poids moléculaire d'à partir de 450-500 k daltons.

15. Agent selon la revendication 11, qui est un complexe de l'acide 2-acétyloxy (benzoïque), le sodium, l'oxygène et le chlore.

16. Agent selon la revendication 15, qui a la formule empirique C₁₈H₁₆O₁₁ClNa.

17. Agent selon la revendication 15 ou 16, qui a un poids moléculaire d'à partir de 400-500 k daltons.

18. Composé pour utilisation en tant que produit pharmaceutique qui est un agent selon l'une des revendications précédentes.

19. Composé pour utilisation en tant qu'agent d'immunomodulation non-spécifique qui est un agent selon l'une quelconque des revendications 1 à 17.

20. Composé pour utilisation en tant qu'agent anti-néoplastique qui est un agent selon l'une quelconque des revendications 1 à 17.

21. Composé pour utilisation en tant qu'agent anti-tumeur qui est un agent selon l'une quelconque des revendications 1 à 17.

22. Utilisation d'un composé qui est un agent selon l'une quelconque des revendications 1 à 23 pour la préparation d'un médicament pour le traitement de tissus bénins.

23. Composition pharmaceutique comprenant un agent selon l'une quelconque des revendications 1 à 17 et un excipient pharmaceutiquement acceptable.

24. Procédé pour préparer un agent antinéoplastique et/ou d'immunomodulation non spécifique comprenant un composant actif qui est un acide ou un sel d'acide lié dans un complexe ensemble avec un métal et un atome d'halogène et présent dans un état excité, lequel procédé comprend les étapes d'incorporer le composant actif dans un mélange d'oxygène et/ou d'ozone, d'halogène et d'un gaz inerte sous pression, diriger le mélange contenant le composant actif à un corps tournant d'une substance capable de vaporisation pour fournir le métal, diriger au corps tournant un laser de façon à effectuer ladite vaporisation, et laisser ledit mélange et la substance vaporisée s'expanser dans un vide et par là former un complexe, ce par quoi le composant actif prend un état excité.

25. Procédé selon la revendication 24, qui inclut l'étape additionnelle d'exciter le complexe avec un second laser.

26. Procédé selon la revendication 24 ou 25, où l'atome d'halogène est le chlore.

27. Procédé selon l'une quelconque des revendications 24 à 26, où le gaz inerte est l'hélium.

28. Procédé selon l'une quelconque des revendications 24 à 27, où le mélange gazeux est sous forme liquide lorsque sous pression.

29. Procédé selon la revendication 28 où le mélange gazeux liquéfié est dirigé par impulsion au corps tournant.

30. Procédé selon l'une quelconque des revendications 24 à 29, où le corps tournant comprend un cristal de ladite substance capable de vaporisation.

## Revendications (Revendications pour l'(les) Etat(s) contractant(s) suivant(s): ES, GR)

1. Procédé pour préparer un agent anti-néoplastique et/ou d'immunomodulation non spécifique comprenant un composant actif qui est un acide ou un sel d'acide lié dans un complexe ensemble avec un métal et un atome d'halogène et présent dans un état excité, lequel procédé comprend les étapes d'incorporer le composant actif dans un mélange d'oxygène et/ou d'ozone, d'halogène et d'un gaz inerte sous pression, diriger le mélange contenant le composant actif à un corps tournant d'une substance capable de vaporisation pour fournir le métal, diriger au corps tournant un laser de façon à effectuer ladite vaporisation, et laisser ledit mélange et ladite substance vaporisée s'expanser dans un vide et par là former un complexe, ce par quoi le composant actif prend un état excité.

2. Procédé selon la revendication 1, qui inclut l'étape additionnelle d'exciter le complexe avec un second laser.

3. Procédé selon la revendication 1 ou 2, où l'halogène est le chlore.

4. Procédé selon l'une quelconque des revendications 1, 2 ou 3, dans lequel le gaz inerte est l'hélium.

5. Procédé selon l'une quelconque des revendications précédentes, dans lequel le mélange gazeux est sous forme liquide lorsque sous pression.

6. Procédé selon la revendication 5 dans lequel le mélange gazeux liquéfié est dirigé en impulsions au corps tournant.

7. Procédé selon l'une quelconque des revendications précédentes, dans lequel le corps tournant comprend un cristal de ladite substance.

8. Procédé selon l'une quelconque des revendications précédentes, dans lequel ledit agent contient au moins deux molécules du composant actif.

9. Procédé selon l'une quelconque des revendications précédentes, dans lequel le composant actif est un acide.

10. Procédé selon la revendication 9, dans lequel le composant actif est un sel partiel de l'acide inorganique ou organique.

11. Procédé selon la revendication 10, dans lequel le complexe contient additionnellement des atomes d'oxygène et d'halogène.

12. Procédé selon la revendication 11, dans lequel l'halogène est le chlore.

13. Procédé selon la revendication 12, dans lequel le complexe acide a la formule
MX/Ac/(O)ₘ
où Ac est le composant actif,
M est un métal fourni par une substance vaporisable,
X est un atome d'halogène ou un mélange de celui-ci, et
m représente le nombre d'atomes d'oxygène et est au moins égal à 1.

14. Procédé selon la revendication 13, où M est un métal alcali.

15. Procédé selon la revendication 13 ou 14, dans lequel M est égal à 3.

16. Procédé selon l'une quelconque des revendications 1 à 7, dans lequel le composant actif est l'acide borique, l'acide citrique, l'acide L-ascorbique, le bicarbonate de sodium, le bicarbonate de potassium, la vitamine A, une semi-pénicilline, l'ibuprofène ou l'acide 2-acétyloxybenzoïque.

17. Procédé selon l'une quelconque des revendications 1 à 7, dans lequel le composant actif est un acide carboxylique aromatique optionnellement substitué par un aryle, ayant adjacent un groupe carboxyle, un groupe acyloxy ou carboxyle optionnellement substitué.

18. Procédé selon la revendication 17, dans lequel ledit agent est un complexe comprenant l'acide 2-acétyloxy(benzoïque), le sodium, l'oxygène et le chlore.

19. Procédé selon la revendication 18, dans lequel ledit complexe a la formule empirique C₁₈H₁₆O₁₁ClK.

20. Procédé selon la revendication 18 ou 19, dans lequel ledit complexe a un poids moléculaire d'à partir de 450-500 k daltons.

21. Procédé selon la revendication 17, dans lequel ledit agent est un complexe comprenant l'acide 2-acétyloxy(benzoïque), le sodium, l'oxygène et le chlore.

22. Procédé selon la revendication 21, dans lequel ledit complexe a la formule empirique C₁₈H₁₆O₁₁ClNa.

23. Procédé selon la revendication 21 ou 22, dans lequel ledit complexe a un poids moléculaire d'à partir de 400-450 k daltons.

24. Procédé selon l'une quelconque des revendications précédentes, dans lequel le composant actif a une activité biologique lorsque à l'état non excité.

25. Procédé selon la revendication 24 dans lequel le composant actif est l'acide borique, l'acide acétique, l'acide citrique, l'acide L-ascorbique, le bicarbonate de sodium, le bicarbonate de potassium, le mono- ou di-sodium ou potassium phosphate, l'hydrogéno-sulfate d'aluminium, un ADN, un ARN, un polypeptide, l'acide glutamique, la vitamine A, la vitamine B ou un sel mono- ou di-phosphate de celle-ci, la vitamine E ou un ester acétate ou succinate de celle-ci, la quinine ou la pénicilline ou un dérivé de celle-ci, l'acide laurique, l'acide stéarique, l'acide dodécylbenzènesulfonique, la chitine, l'acide lactique, un polysaccharide, un opiate, l'ibuprofène, l'acide 2-acétylbenzoïque ou un sel de celui-ci.

26. Procédé selon la revendication 25, dans lequel le composant actif est l'acide borique, l'acide citrique, l'acide L-ascorbique, le bicarbonate de sodium, le bicarbonate de potassium, la vitamine A, une semipénicilline, l'acide laurique, l'acide stéarique, l'acide dodécylbenzènesulfonique, l'ibuprofène ou l'acide 2-acétyloxybenzoïque.
